# EUROPÄISCHE PATENTANMELDUNG

(11) **EP 4 790 413 A1**
(43) Veröffentlichungstag der Anmeldung: **12.08.2026**
(21) Anmeldenummer: 26157335.6
(22) Anmeldetag: 09.02.2026
(51) Int. Cl.: G01N 33/28, G01N 27/02

(54) **SONDE UND MESSSYSTEM FÜR EIN ZUMINDEST TEILWEISE IN ÖL BEFINDLICHES ELEKTRISCHES BETRIEBSMITTEL**

(30) Priorität: 11.02.2025 DE 102025105068
(71) Anmelder: E.ON SE, 45131 Essen (DE)
(72) Erfinder: Kumar, Rajkiran, 80992 München (DE); Valentine, Darren, 80992 München (DE); Fecher, Florian, 80992 München (DE); Hernando Armengol, Alejandro, 80634 München (DE)
(74) Vertreter: Gunzelmann, Rainer

(57) **Zusammenfassung**

Es wird ein Sonde (10) für ein zumindest teilweise in Öl befindliches elektrisches Betriebsmittel beschrieben, wobei die Sonde (10) eine Leiterplatte (11) mit einem oder mehreren Sensorelement(en) (A1 - A6), und einen auf der Leiterplatte integrierten Multiplexer und/oder Demultiplexer (15), der zur Steuerung eines Signalflusses von und zu den Sensorelementen (A1 - A6) konfiguriert ist, umfasst. Ferner wird ein Messsystem umfassend eine Sonde (10) und eine Signaleinheit (300), die dazu eingerichtet ist, ein elektrisches Signal an den Multiplexer und/oder Demultiplexer (15) zu senden und/oder ein elektrisches Signal von dem Multiplexer und/oder Demultiplexer (15) zu empfangen, beschrieben.

## Beschreibung

### TECHNISCHES GEBIET

Die vorliegende Offenbarung betrifft eine Sonde für ein zumindest teilweise in Öl befindliches elektrisches Betriebsmittel und ein Messystem mit einer Sonde und einer Signaleinheit.

### HINTERGRUND

Bei zumindest teilweise in Öl befindlichen elektrischen Betriebsmitteln, wie beispielsweise Öltransformatoren, unterliegt das Öl einer Alterung. Diese Alterung kann die Leistung, Zuverlässigkeit und Lebensdauer des Transformators beeinträchtigen. Bei stark gealtertem Öl kann ein vollständiger Ölwechsel erforderlich werden.

Um den Zustand von Transformatorenöl zu beurteilen, wird üblicherweise eine Probe entnommen und zur detaillierten Analyse in ein Labor geschickt. Dieser Ansatz liefert Einblicke in die Qualität und den Zustand des Öls, insbesondere durch die Messung gelöster Gase wie Wasserstoff, Methan, Ethylen und Acetylen sowie des Feuchtigkeitsgehalts, die auf thermische oder elektrische Fehler und eine mögliche Verschlechterung des Transformators hinweisen können. Allerdings bringt diese Methode Verzögerungen durch den Transport und die Laboruntersuchung mit sich und erfordert den Einsatz von Personal zur Probenentnahme vor Ort am Transformator.

Alternativ kann eine Analyse direkt vor Ort mithilfe von Zustandsüberwachungssystemen durchgeführt werden. Diese Systeme automatisieren die Entnahme von Ölproben und nutzen Technologien, wie Sensoren und Detektionstechniken, um Eigenschaften wie die Konzentration gelöster Gase und Feuchtigkeitsgehalt zu bewerten. Obwohl dies häufigere Messungen ermöglicht, führen die komplexen Technologien, die sowohl für die Probenentnahme als auch für die Analyse erforderlich sind, zu erheblich höherem Aufwand. Die Abhängigkeit von spezialisierten Sensoren für verschiedene Messungen treibt den Messauwand zusätzlich in die Höhe, was solche Systeme für einige Transformatorbetreiber nicht einsetzbar macht.

### ABRISS DER OFFENBARUNG

Der vorliegenden Offenbarung liegt die Aufgabe zugrunde, eine Sonde und ein Messystem für ein zumindest teilweise in Öl befindliche elektrische Betriebsmittel bereitzustellen, welche einen Messaufwand vereinfachen und verkürzen.

Zur Lösung dieser Aufgabe wird eine Sonde für ein zumindest teilweise in Öl befindliches elektrisches Betriebsmittel (eine zumindest teilweise in Öl eingetauchte Energieanlage) vorgeschlagen, welche eine Leiterplatte mit einem oder mehreren Sensorelement(en), und einen auf der Leiterplatte integrierten Multiplexer und/oder Demultiplexer, der zur Steuerung eines Signalflusses von und zu den Sensorelementen konfiguriert ist, umfasst.

Bei dem elektrisches Betriebsmittel kann es sich beispielsweise um einen Öltransformator, einen Ölschalter, eine ölgefüllte Schaltanlage, einen ölgefüllten Strom- und Spannungswandler, einen ölgefüllten Kondensator, eine ölgefüllte Drosselspule, einen ölgefüllten Reaktor, eine ölgefüllte Durchführung, ein ölisoliertes Kabel, einen ölgefüllten Laststufenschalter, einen ölgefüllten Motor, einen ölgefüllten Generator, einen ölgefüllten Leistungssteller, einen ölgefüllten Thyristor oder einen ölgefüllten Lichtbogenofen handeln.

Das Öl dient zur elektrischen Isolation und Wärmeabfuhr des elektrischen Betriebsmittels. Bei dem Öl kann es sich beispielsweise um ein Mineralöl oder ein synthetisches Esteröl handeln.

Bei der Leiterplatte, beispielsweise einem Printed Circuit Board (PCB), mit einer oder mehreren geätzten Schichten, die Sensorelemente bilden, kann es sich um ein elektronisches Bauteil handeln, bei dem die Leiterplatte nicht nur als Träger für elektrische Verbindungen der Sensorelemente dient, sondern auch die Sensorik direkt in ihre Schichten integriert. Die geätzten Schichten beziehen sich auf speziell bearbeitete Bereiche der Leiterplatte, bei denen Material präzise entfernt wurde, um Leitbahnen, Kontaktflächen und Sensorelemente zu formen.

Es können auch verschiedene andere Verfahren zur Herstellung von Sensorelementen, wie Nanomaterial-basierten Sensoren, die sich im Wesentlichen in top-down- und bottom-up-Ansätze unterteilen, verwendet werden. Bei der Lithografie als top-down-Methode wird das gewünschte Material präzise auf einem Substrat strukturiert. Dazu lässt sich beispielsweise die Photolithografie einsetzen, bei der zunächst eine fotoempfindliche Schicht (Photoresist) auf das Substrat aufgetragen und anschließend durch UV-Belichtung mittels einer Maske definiert wird. Nach dem Entwickeln bleiben die gewünschten Bereiche erhalten, während das übrige Material durch Ätzen entfernt werden kann. Eine weitere Variante ist die Elektronenstrahllithografie, die anstelle von UV-Licht fokussierte Elektronenstrahlen nutzt und dadurch eine noch höhere Auflösung bei der Mustererzeugung ermöglicht.

Im Gegensatz dazu beruht die Dielektrophorese (DEP) auf einem bottom-up-Ansatz. Hier werden Nanomaterialien in einer Suspension zubereitet und durch Anlegen eines Wechselstroms (AC) zwischen Mikroelektroden auf einem Substrat in Bereiche hoher elektrischer Feldstärke gezogen. Sobald die Nanopartikel auf diese Weise fixiert sind, wird das flüssige Medium verdampft, sodass die Teilchen an Ort und Stelle verbleiben und so einen Teil des Sensors bilden.

Daneben existieren weitere Synthesemethoden, etwa die chemische Gasphasenabscheidung (Chemical Vapor Deposition, CVD), mit der sich hochqualitative Kohlenstoffnanoröhren (CNTs) oder Graphen bei hohen Temperaturen aus gasförmigen Ausgangsmaterialien auf dem Substrat ablagern lassen. Eine andere Möglichkeit ist das Sol-Gel-Verfahren, bei dem eine kolloidale Suspension (Sol) zu einem Gel überführt wird, was sich insbesondere für die Herstellung von Metalloxid-Nanostrukturen anbietet.

Bei der späteren Integration der so gewonnenen Nanomaterialien werden Erkennungselemente (z. B. Antikörper oder DNA) an das Nanomaterial gekoppelt und mit passenden Signalwandlern verbunden (z. B. Elektroden für elektrochemische Sensoren). Für die Charakterisierung der hergestellten Nanostrukturen kommen unter anderem Verfahren wie die Rasterelektronenmikroskopie (Scanning Electron Microscopy, SEM) oder die Rasterkraftmikroskopie (Atomic Force Microscopy, AFM) zum Einsatz, um Morphologie und Oberflächeneigenschaften genau zu analysieren. Zur Herstellung der Sensorelemente kann insbesondere Folgendes verwendet werden:
- verschiedene Verfahren wie Elektronenstrahllithografie und Röntgenlithografie
- unterschiedliche Substrat- und Katalysatormaterialien wie Palladium (Pd), Nickel (Ni), Zinnoxid usw.
- verschiedene Strukturorientierungen (vertikal, horizontal)
- unterschiedliche Längen (von 1 m bis 1 km)
- verschiedene Bindematerialien zur Verbindung der Elemente auf der Leiterplatte, wie Gold, Kupfer, Platin usw.

Die Sensorelemente können dazu eingerichtet sein, gelöste Gase, einen Feuchtigkeitsgehalt, eine Säurezahl, dielektrische Eigenschaften und/oder eine Furankonzentration des bzw. in dem Öl zu messen. Ferner kann jede andere relevante Öleigenschaft gemessen werden.

Der auf der Leiterplatte integrierte Multiplexer und/oder Demultiplexer dient einer effizienten Steuerung des Signalflusses von und zu den Sensorelementen. Der Multiplexer ist ein elektronisches Bauteil, das mehrere Eingangssignale aufnehmen und sie über eine einzige Leitung an eine Ausgabeeinheit weiterleiten kann. Dieses Prinzip ermöglicht es, die Daten mehrerer Sensoren über einen gemeinsamen Kommunikationskanal zu übertragen, was die Anzahl der benötigten Verbindungen reduziert und die Komplexität des Schaltungsdesigns vereinfacht.

Der Demultiplexer erfüllt die entgegengesetzte Funktion. Er empfängt ein einzelnes Eingangssignal und verteilt es auf mehrere Ausgangsleitungen. In Bezug auf die Sensorelemente kann der Demultiplexer verwendet werden, um Steuersignale oder Betriebsspannung selektiv an bestimmte Sensoren oder Sensorgruppen zu senden. Dies erlaubt eine gezielte Ansteuerung der Sensoren, sodass nur die gewünschten Sensoren zu einem bestimmten Zeitpunkt aktiviert oder ausgelesen werden. So kann der Multiplexer und/oder Demultiplexer zum sequenziellen oder gleichzeitigen Senden und/oder Empfangen von elektrischen Signalen zu bzw. von den Sensorelementen eingerichtet sein.

Die Integration von Multiplexer und/oder Demultiplexer direkt auf der Leiterplatte bietet mehrere Vorteile. Sie ermöglicht eine kompakte Bauweise, da zusätzliche externe Komponenten und Verbindungen entfallen. Darüber hinaus verbessert sie die Signalqualität, da kürzere Signalwege zu geringeren Verlusten und weniger Störungen führen. Die Steuerung dieser Bauteile kann durch eine zentrale Verarbeitungseinheit erfolgen, die mittels Steuersignalen bestimmt, welche Sensoren aktiviert werden und welche Signale weitergeleitet oder empfangen werden sollen. Durch die Verwendung von Multiplexern und Demultiplexern kann das System effizient skalieren, indem bei Bedarf zusätzliche Sensoren hinzugefügt werden, ohne die grundlegende Architektur zu verändern. Dies ist besonders nützlich in Anwendungen, bei denen viele Sensorelemente auf begrenztem Raum untergebracht werden müssen, wie Sensoren, die über enge Öffnungen in Ölgefäße eingeführt werden müssen.

Auf der Leiterplatte kann auch eine Vielzahl von Multiplexern und/oder Demultiplexern vorgesehen sein.

Die Sensorelemente können chemische Katalysatoren und/oder Mikroreaktoren und/oder chemische Zusammensetzungen umfassen. Die chemischen Katalysatoren können so konfiguriert sein, dass sie mit einem im Öl gelösten Gas reagieren, um ein elektrisches Signal zu erzeugen, das ein Indikator für eine Anwesenheit und/oder eine Konzentration des Gases ist. Dies bedeutet, dass die Sensoren auf chemischer Ebene arbeiten, indem sie spezifische Reaktionen mit Zielgasen eingehen, die im Öl vorhanden sind. Chemische Katalysatoren sind Substanzen, die eine chemische Reaktion ermöglichen oder beschleunigen, ohne dabei selbst verbraucht zu werden. In diesem Kontext sind sie so gestaltet, dass sie gezielt mit bestimmten Gasen wie Wasserstoff oder Feuchtigkeit reagieren, die im Transformatoröl gelöst sein können. Wenn das Zielgas mit dem Katalysator in Kontakt kommt, findet eine spezifische chemische Reaktion statt. Diese Reaktion führt zu einer Veränderung in den physikalischen oder chemischen Eigenschaften des Katalysators oder des umgebenden Materials. Diese Veränderungen können elektrische Eigenschaften wie Leitfähigkeit, Widerstand oder elektrisches Potenzial beeinflussen. Die Sensorelemente erfassen diese Veränderungen und wandeln sie in elektrische Signale um. Diese Signale sind direkt proportional zur Anwesenheit und oft auch zur Konzentration des spezifischen Gases im Öl. Durch geeignete Kalibrierung und Signalverarbeitung kann die genaue Konzentration des Gases bestimmt werden. Die Integration der chemischen Katalysatoren in die Sensorelemente der Leiterplatte ermöglicht eine kompakte und effiziente Bauweise. Da die Reaktion direkt auf der Oberfläche der Leiterplatte stattfindet, können die entstehenden Signale schnell erfasst und verarbeitet werden. Dies ist besonders vorteilhaft für Echtzeitüberwachungssysteme, bei denen schnelle Reaktionszeiten entscheidend sind.

Die Sensorelemente können aus einer chemischen Zusammensetzung bestehen. Diese chemische Zusammensetzung kann so konfiguriert werden, dass sie mit einem gelösten Fehlergas und/oder dem Transformatorenöl interagiert. Die Interaktion kann schwach oder gering sein. Durch diese Interaktion können sich die physikalischen, chemischen und/oder elektrochemischen Eigenschaften der chemischen Zusammensetzung verändern. Diese Veränderungen können elektrische Eigenschaften wie Leitfähigkeit, Widerstand oder elektrisches Potenzial beeinflussen. Dadurch ändern die Sensorelemente ihre Reaktion auf angelegte elektromagnetische Signale. Diese Veränderungen in den Antwortsignalen können auf das Vorhandensein und häufig auch auf die Konzentration des jeweiligen Gases im Öl hinweisen.

Durch geeignete Kalibrierung und Signalverarbeitung kann die genaue Gaskonzentration ermittelt werden. Die Integration der chemischen Zusammensetzung in die Sensorelemente der Leiterplatte ermöglicht ein kompaktes und effizientes Design. Da die Interaktionen direkt auf der Oberfläche der Leiterplatte stattfinden, können die resultierenden Signale schnell erfasst und verarbeitet werden. Dies ist besonders vorteilhaft für EchtzeitÜberwachungssysteme, bei denen schnelle Reaktionszeiten entscheidend sind.

Die Verwendung solcher Sensorelemente ist insbesondere vorteilhaft für die Überwachung von ölgetauchten elektrischen Betriebsmitteln wie Transformatoren. Eine erhöhte Konzentration bestimmter Gase im Öl kann auf Probleme wie Überhitzung, Isolationsfehler oder andere Betriebsstörungen hinweisen. Durch die frühzeitige Detektion dieser Gase können Wartungsmaßnahmen rechtzeitig eingeleitet werden, um Schäden zu verhindern und die Lebensdauer der elektrischen Betriebsmittel zu verlängern.

Die Mikroreaktoren können so konfiguriert sein, dass sie mit einem im Öl gelösten Gas interagieren, um ein elektrisches Signal zu erzeugen, das auf eine Anwesenheit und/oder eine Konzentration des Gases hinweist. Mikroreaktoren sind kleine Reaktionsräume auf der Leiterplatte, die gestaltet sind, um die Wechselwirkung zwischen dem gelösten Gas und den Sensormaterialien zu maximieren. Durch ihre geringe Größe bieten sie eine große Oberfläche relativ zum Volumen, was die Effizienz der chemischen oder physikalischen Reaktionen erhöht. In diesen Mikroreaktoren kann das gelöste Gas entweder eine chemische Reaktion mit einem Reagenz eingehen oder physikalisch an einer sensitiven Oberfläche adsorbiert werden. Diese Wechselwirkung führt zu einer messbaren Änderung einer elektrischen Eigenschaft, wie beispielsweise des elektrischen Widerstands, der Kapazität oder des Potenzials. Diese Änderung wird von den Sensorelementen erfasst und in ein elektrisches Signal umgewandelt. Dieses Signal ist proportional zur Konzentration des Gases und kann somit als Indikator für dessen Anwesenheit und Menge dienen. Die Konfiguration der Mikroreaktoren kann an verschiedene Gase angepasst werden, indem spezifische Materialien oder Beschichtungen verwendet werden, die selektiv mit dem Zielgas interagieren. Dies ermöglicht eine hohe Sensitivität und Selektivität bei der Gasdetektion. Die Integration dieser Mikroreaktoren auf der Leiterplatte ermöglicht eine kompakte Bauweise und kurze Signalwege, was die Reaktionszeit verkürzt und die Signalqualität verbessert. Durch die Verwendung von Mikroreaktoren in den Sensorelementen können wichtige Gase wie Wasserstoff oder Feuchtigkeit, die im Transformatoröl gelöst sind, effektiv überwacht werden. Eine erhöhte Konzentration dieser Gase kann auf potenzielle Probleme oder Alterungsprozesse im Transformator hinweisen. Die Fähigkeit, diese Gase schnell und präzise zu detektieren, trägt zur vorbeugenden Instandhaltung und zur Verlängerung der Lebensdauer des elektrischen Betriebsmittels bei.

Die Sensorelemente können verschiedene geometrische Formen aufweisen, was auf sowohl funktionalen als auch designtechnischen Überlegungen basieren kann. Unterschiedliche geometrische Formen ermöglichen es, die Eigenschaften der Sensoren wie Empfindlichkeit, Selektivität und Reaktionszeit an die spezifischen Anforderungen der Anwendung anzupassen. So kann beispielsweise die Vergrößerung der Oberfläche eines Sensorelements durch eine gezielte geometrische Gestaltung die Interaktion mit dem zu detektierenden Gas verbessern, was die Sensitivität des Sensors erhöht. Flächige, planare Formen erleichtern die Integration der Sensorelemente in die Leiterplatte und ermöglichen eine gleichmäßige Verteilung elektrischer Felder, was zu präziseren Messungen führt. Dreidimensionale Strukturen wie Mikrokanäle oder Kavitäten können genutzt werden, um den Fluss von Gasen oder Flüssigkeiten zu steuern und die Reaktionszeiten zu verkürzen, indem sie den Transport des Analyten zum aktiven Bereich des Sensors beschleunigen. Auch spezielle Formen wie spiralförmige oder meandrierende Strukturen können eingesetzt werden, um bestimmte elektrische Eigenschaften wie Induktivität oder Kapazität zu beeinflussen, die für bestimmte Messprinzipien relevant sind. Die Variation der geometrischen Formen ermöglicht es zudem, mehrere Sensorelemente auf begrenztem Raum effizient anzuordnen, was die Funktionalität und Kompaktheit des Gesamtsystems verbessert. Die gezielte Gestaltung der geometrischen Form der Sensorelemente ist daher ein wichtiger Faktor bei der Entwicklung von Sensoren. Sie erlaubt es, die physikalischen und chemischen Eigenschaften der Sensoren zu optimieren und an die spezifischen Anforderungen der Anwendung anzupassen, wodurch die Leistungsfähigkeit und Zuverlässigkeit des Sensorsystems insgesamt gesteigert werden.

Die Sensorelemente können so eingerichtet sein, dass sie miteinander über ein Telekommunikationsprotokoll wie Transmission Control Protocol/Internet Protocol (TCP/IP) oder User Datagram Protocol (UDP) kommunizieren. Dazu kann jedes Sensorelement mit einer eigenen Netzwerkadresse ausgestattet sein und Daten werden über ein gemeinsames Netzwerk auf der Leiterplatte ausgetauscht. Durch die Verwendung von standardisierten Protokollen wie TCP/IP oder UDP wird eine zuverlässige und effiziente Datenübertragung ermöglicht. Dies erleichtert die Integration der Sensorelemente in bestehende Netzwerkinfrastrukturen und erlaubt eine skalierbare und flexible Systemarchitektur. Die Kommunikation über diese Protokolle ermöglicht es den Sensorelementen, Informationen in Echtzeit zu teilen, Synchronisationen durchzuführen und auf gemeinsame Ereignisse zu reagieren. Zudem kann durch die Netzwerkfähigkeit eine zentrale Überwachung und Steuerung der Sensorelemente erfolgen, was die Wartung und Verwaltung des Gesamtsystems vereinfacht und die Gesamteffizienz steigert. Die Sensorelemente können von dem Multiplexer/Demultiplexer über das Netzwerk gesteuert werden.

Die Leiterplatte mit integrierten Sensorelementen kann mit weiteren elektronischen Bauteilen bestückt sein, wie Mikrocontrollern, Signalaufbereitungs- und Kommunikationseinheiten. Dadurch kann ein komplettes Sensorsystem bereitgestellt werden, das kompakt und effizient ist.

Die Sonde kann ferner eine eingebettete Einheit, eine anwendungsspezifisch integrierte Schaltung, ASIC, eine System-on-a-Chip, SoC, elektronische Einheit, einen Mikrocontroller, MCU, einen Digital-Analog-Wandler, DAC, und/oder einen Analog-Digital-Wandler, ADC, der/die auf der Leiterplatte in elektrischer Verbindung mit dem Multiplexer und/oder Demultiplexer und den Sensorelementen angeordnet ist/sind, umfassen.

Dies bedeutet, dass die Sonde mit zusätzlichen elektronischen Komponenten ausgestattet sein kann, die ihre Funktionalität erweitern und die Verarbeitung der von den Sensorelementen erzeugten Signale ermöglichen. Die eingebettete Einheit oder der Mikrocontroller dient als zentrales Steuerungselement, das die Daten von den Sensorelementen sammelt, verarbeitet und an andere Systeme oder Benutzer weiterleitet. Der Mikrocontroller ist ein kleiner Computer auf einem Chip, der Prozessor, Speicher und Ein-/Ausgabe-Peripherie integriert. Er kann Programme ausführen, die speziell für die Steuerung der Sensorfunktionen und die Datenverarbeitung entwickelt wurden. Der ASIC ist eine maßgeschneiderte integrierte Schaltung, die für eine spezifische Anwendung oder Funktion entwickelt wurde. In diesem Kontext kann ein ASIC komplexe Signalverarbeitungsaufgaben übernehmen, die Effizienz steigern und den Platzbedarf reduzieren, da mehrere Funktionen in einem einzigen Chip integriert sind. Der SoC geht noch einen Schritt weiter, indem es alle notwendigen elektronischen Komponenten eines Systems auf einem einzigen Chip vereint, einschließlich Prozessor, Speicher, Schnittstellen und oft auch analoge Komponenten wie DACs und ADCs. Die Digital-Analog-Wandler (DAC) und Analog-Digital-Wandler (ADC) sind wichtig für die Kommunikation zwischen den analogen Sensorelementen und den digitalen Verarbeitungseinheiten. Die ADCs wandeln die analogen Signale der Sensoren in digitale Daten um, die vom Mikrocontroller oder ASIC verarbeitet werden können. Umgekehrt ermöglichen DACs die Ausgabe von analogen Signalen, falls dies für Steuerungszwecke oder für die Ansteuerung anderer analoger Komponenten erforderlich ist.

Durch die Integration dieser Komponenten auf der Leiterplatte und ihre elektrische Verbindung mit dem Multiplexer und/oder Demultiplexer sowie den Sensorelementen entsteht ein hochintegriertes System. Der Multiplexer und Demultiplexer steuern den Signalfluss zwischen den Sensorelementen und den Verarbeitungseinheiten, sodass Daten effizient gesammelt und verarbeitet werden können. Diese Architektur ermöglicht eine schnelle, zuverlässige und effiziente Datenerfassung und -verarbeitung innerhalb der Sonde, was für präzise Messungen und Echtzeitüberwachung vorteilhaft ist.

Die eingebettete Einheit, die anwendungsspezifische integrierte Schaltung (ASIC), die System-on-a-Chip (SoC) elektronische Einheit, der Mikrocontroller (MCU), der Digital-Analog-Wandler (DAC) und der Analog-Digital-Wandler (ADC) können einzeln oder gemeinsam dazu beitragen, Rauschen zu reduzieren und eine Signalübertragung sowie einen Empfang zu verbessern. Der Mikrocontroller oder die SoC-Einheit übernimmt die Steuerung der Signalverarbeitung und kann durch implementierte Algorithmen zur digitalen Filterung und Rauschunterdrückung beitragen. Eine ASIC kann dafür ausgelegt sein, Signalverarbeitungsaufgaben mit hoher Effizienz und geringer Verzögerung durchzuführen, was die Qualität der Datenübertragung verbessert. Der Analog-Digital-Wandler (ADC) spielt eine wichtige Rolle bei der Umwandlung der analogen Signale der Sensorelemente in digitale Signale. Ein hochwertiger ADC mit hoher Auflösung und schneller Abtastrate kann das Signal-Rausch-Verhältnis verbessern, indem er die Genauigkeit der Signalumwandlung erhöht und quantisierungsbedingtes Rauschen minimiert. Der Digital-Analog-Wandler (DAC) hingegen ermöglicht die präzise Umwandlung von digitalen Signalen in analoge Signale, was wichtig ist, wenn analoge Steuersignale oder Feedback-Schleifen benötigt werden. Durch die Integration dieser Komponenten auf der Leiterplatte kann die Signalpfadlänge minimiert werden, was elektromagnetische Störungen reduziert. Zudem können abgeschirmte Leitungen und geeignete Layout-Techniken eingesetzt werden, um Übersprechen und andere Formen von elektrischem Rauschen zu verringern. Die Abstimmung der Komponenten ermöglicht eine effiziente Signalverarbeitung in Echtzeit, wobei Fehlerkorrekturmechanismen und Signalmodulationstechniken eingesetzt werden können, um die Robustheit der Datenübertragung zu erhöhen.

Die Sonde kann ferner ein Kommunikationsmodul in Kommunikationsverbindung mit einem der eingebetteten Einheit, der anwendungsspezifisch integrierten Schaltung, ASIC, der System-on-a-Chip, SoC, elektronischen Einheit, dem Mikrocontroller, MCU, dem Digital-Analog-Wandler, DAC, und/oder dem Analog-Digital-Wandler, ADC, umfassen. Das Kommunikationsmodul kann auf der Leiterplatte angeordnet und dazu eingerichtet sein, Messwerte der Sensorelemente drahtlos an eine externe Empfangseinheit oder in eine Daten-Cloud zu senden. Dazu kann die Sonde mit einer Schnittstelle ausgestattet sein, die es ermöglicht, die erfassten Daten der Sensorelemente effizient nach außen zu senden. Indem das Kommunikationsmodul mit den zentralen Verarbeitungseinheiten wie dem Mikrocontroller oder der ASIC verbunden ist, können die Messwerte in Echtzeit oder in definierten Intervallen an externe Systeme übermittelt werden. Dies ist vorteilhaft für Anwendungen, bei denen eine kontinuierliche Überwachung erforderlich ist oder die Daten für weitere Analysen oder Steuerungsaufgaben genutzt werden sollen. Das Kommunikationsmodul kann verschiedene Formen annehmen, je nach den spezifischen Anforderungen der Anwendung. Es kann sich um ein kabelgebundenes Interface handeln, wie beispielsweise UART, SPI oder I²C, oder um eine drahtlose Verbindung mittels LTE, Bluetooth, Wi-Fi oder anderen Funktechnologien. Die Wahl des Kommunikationsprotokolls hängt von Faktoren wie der erforderlichen Datenrate, Reichweite, Energieeffizienz und den Umgebungsbedingungen ab.

Durch die Integration des Kommunikationsmoduls auf der Leiterplatte wird die Sonde zu einem intelligenten Gerät, das nicht nur Daten erfasst, sondern auch aktiv mit anderen Systemen kommunizieren kann. Die externe Empfangseinheit, die die gesendeten Messwerte erhält, kann diese Daten verarbeiten, anzeigen oder für langfristige Überwachungszwecke speichern. Dies ermöglicht eine verbesserte Diagnose und Wartung der elektrischen Betriebsmittel, da potenzielle Probleme frühzeitig erkannt und entsprechende Maßnahmen ergriffen werden können. Insgesamt trägt das Kommunikationsmodul dazu bei, die Effizienz und Zuverlässigkeit des Überwachungssystems zu erhöhen, indem es eine nahtlose Integration in bestehende Netzwerk- und Steuerungsstrukturen ermöglicht.

Die Sonde kann ferner zusätzlichen zu den Sensorelementen einen Sensor umfassen, der mit dem Multiplexer und/oder Demultiplexer elektrisch verbunden und dazu eingerichtet ist, eine Temperatur, eine Viskosität, eine Vibration und/oder einen Durchfluss des Öls zu messen. Es kann auch eine Vielzahl von Sensoren vorgesehen sein. Der Sensor kann in der Leiterplatte integriert sein. So kann die Sonde nicht nur in der Lage sein, chemische Eigenschaften des Öls zu überwachen, sondern auch wichtige physikalische Parameter erfassen. Der in die Sonde integrierte Sensor erweitert die Funktionalität des Systems, indem er zusätzliche Daten liefert, die für die Bewertung des Zustands des ölgetauchten elektrischen Betriebsmittels relevant sind. Die Messung der Temperatur des Öls ist wichtig, da Temperaturänderungen auf Betriebszustände wie Überhitzung oder ineffiziente Kühlung hinweisen können. Eine genaue Überwachung der Öltemperatur hilft dabei, thermische Belastungen frühzeitig zu erkennen und entsprechende Maßnahmen einzuleiten. Die Viskosität des Öls ist ein Indikator für dessen Zustand und Qualität. Veränderungen in der Viskosität können auf Alterungsprozesse, Verunreinigungen oder den Abbau von Additiven hindeuten. Durch die kontinuierliche Messung der Viskosität können Wartungsintervalle optimiert und die Effizienz des elektrischen Betriebsmittels gesteigert werden. Die Erfassung von Vibrationen liefert wichtige Informationen über mechanische Einflüsse oder mögliche Schäden innerhalb des Systems. Ungewöhnliche Vibrationen können auf Lagerprobleme, Unwuchten oder andere mechanische Defekte hinweisen. Die frühzeitige Detektion solcher Anomalien trägt dazu bei, Ausfälle zu verhindern und die Lebensdauer des elektrischen Betriebsmittels zu verlängern. Die Messung des Durchflusses des Öls ist ebenfalls vorteilhaft. Ein ausreichender Ölfluss gewährleistet die effektive Wärmeabfuhr und die Schmierung beweglicher Teile. Veränderungen im Durchfluss können auf Verstopfungen, Leckagen oder Pumpenprobleme hindeuten. Durch die Überwachung des Ölflusses kann beispielsweise sichergestellt werden, dass ein Kühlsystem ordnungsgemäß funktioniert und das elektrische Betriebsmittel unter optimalen Bedingungen arbeitet.

Die Integration dieses Sensors bzw. der Vielzahl von Sensoren in das bestehende System der Sonde ermöglicht eine effiziente Datenverwaltung. Durch die elektrische Verbindung mit dem Multiplexer und/oder Demultiplexer können die Signale des Sensors gemeinsam mit den Daten der Sensorelemente verarbeitet werden. Dies reduziert die Komplexität der Schaltung und ermöglicht eine zentrale Steuerung und Auswertung aller relevanten Messgrößen. Insgesamt erweitert der zusätzliche Sensor die diagnostischen Fähigkeiten der Sonde und trägt zu einer umfassenderen Überwachung und Wartung des elektrischen Betriebsmittels bei.

Die Sonde kann ferner eine Temperatursteuerungseinheit umfassen, die dazu eingerichtet ist, eine Temperatur der Sensorelemente zu steuern. Dazu kann die Sonde über eine integrierte Vorrichtung verfügt, die kontinuierlich die Temperatur der Sensoren überwacht und aktiv steuert, um optimale Betriebsbedingungen sicherzustellen. Die Kontrolle der Temperatur ist vorteilhaft, da die Leistungsfähigkeit der Sensorelemente von ihrer Betriebstemperatur abhängt. Schwankungen in der Temperatur können die Empfindlichkeit, Genauigkeit und Lebensdauer der Sensoren beeinträchtigen. Durch die Aufrechterhaltung einer stabilen Temperatur wird sichergestellt, dass die Sensoren konsistente und zuverlässige Messungen liefern. Die Temperatursteuerungseinheit kann in der Leiterplatte integriert sein und mit einem Mikrocontroller der Sonde verbunden sein. Die Temperatursteuerungseinheit kann verschiedene Technologien nutzen, wie zum Beispiel Heizelemente, Kühlkörper oder thermoelektrische Module, um die Temperatur entsprechend anzupassen. Sie arbeitet in Verbindung mit den elektronischen Komponenten der Sonde, wie dem Mikrocontroller oder der eingebetteten Einheit, die die Steuerungsalgorithmen implementieren. Insgesamt trägt die Temperatursteuerungseinheit dazu bei, die Effizienz und Genauigkeit der Sonde unter unterschiedlichen Umgebungsbedingungen zu erhöhen, indem sie die Sensorelemente innerhalb ihres optimalen Temperaturbereichs hält.

Die Sonde kann ferner einen Filterabschnitt, insbesondere eine Membran, umfassen der dazu eingerichtet ist, Partikel aus dem Öl zu filtern und/oder Gas aus dem Öl zu extrahieren. Dazu kann die Sonde mit einer speziellen Membran ausgestattet ist, die die Reinheit des Öls verbessert und die Effizienz der Gasdetektion erhöht. Die Membran dient als Barriere, die feste Partikel, Verunreinigungen oder Ablagerungen zurückhält, welche die Sensorelemente beeinträchtigen oder deren Lebensdauer verkürzen könnten. Durch das Entfernen dieser Partikel wird sichergestellt, dass das Öl in sauberem Zustand zu den Sensorelementen gelangt, was die Genauigkeit der Messungen erhöht.

Des Weiteren kann die Membran so gestaltet sein, dass sie gasdurchlässig ist und somit die Extraktion von im Öl gelösten Gasen ermöglicht. Dies ist vorteilhaft für die effektive Detektion von Gasen wie Wasserstoff oder Feuchtigkeit, die Indikatoren für den Zustand des elektrischen Betriebsmittels sind. Die Membran kann selektiv bestimmte Gase passieren lassen, wodurch die Konzentration dieser Gase an den Sensorelementen erhöht wird und eine schnellere und empfindlichere Messung ermöglicht wird. Durch die Kombination von Partikelfiltration und Gasextraktion trägt der Filterabschnitt zur Verbesserung der Leistungsfähigkeit der Sonde bei. Er schützt die internen Komponenten vor Verschmutzung und Verschleiß und stellt sicher, dass die Sensorelemente unter optimalen Bedingungen arbeiten. Dies führt zu zuverlässigeren Daten und ermöglicht eine effektivere Überwachung und Wartung von ölgetauchten elektrischen Betriebsmitteln.

Die Sonde kann ferner ein die Leiterplatte umgebendes Gehäuse umfassen, wobei der Filterabschnitt in dem Gehäuse integriert ist. Dias Gehäuse dient dazu, die empfindliche Elektronik der Leiterplatte vor äußeren Einflüssen wie mechanischen Belastungen, Feuchtigkeit oder Verschmutzungen zu schützen. Durch die Integration des Filterabschnitts in das Gehäuse wird ein kompakter Aufbau erreicht, der die Funktionalität der Sonde optimiert. Der Filterabschnitt ermöglicht es, Partikel aus dem Öl zu entfernen und Gase aus dem Öl zu extrahieren, bevor sie die Sensorelemente erreichen. Dies gewährleistet, dass die Sensorelemente unter idealen Bedingungen arbeiten können, da sie vor Verunreinigungen geschützt sind und eine präzise Messung der im Öl gelösten Gase ermöglichen. Das Gehäuse mit integriertem Filter trägt somit zur Leistungsfähigkeit und Langlebigkeit der Sonde bei, indem es sowohl den Schutz der elektronischen Komponenten sicherstellt als auch die Effektivität der Sensorik verbessert.

Die Sonde kann auch eine Schutzschicht umfassen, die zumindest den Multiplexer und/oder Demultiplexer umgibt. Diese Schutzschicht dient dazu, die empfindlichen elektronischen Komponenten vor äußeren Einflüssen zu schützen, die ihre Funktion beeinträchtigen könnten. In Anwendungen, bei denen die Sonde in Öl oder anderen potenziell aggressiven Medien eingesetzt wird, ist es besonders wichtig, die elektronischen Bauteile vor Feuchtigkeit, chemischen Substanzen, Temperaturschwankungen und mechanischen Beschädigungen zu bewahren. Die Schutzschicht kann aus Materialien bestehen, die eine Barriere gegen solche Einflüsse bilden, wie zum Beispiel spezielle Lacke, Harze oder Vergussmassen, die elektrisch isolierend sind und gleichzeitig die Wärmeabfuhr nicht behindern. Durch die Umschließung des Multiplexers/Demultiplexers wird sichergestellt, dass diese kritischen Komponenten zuverlässig arbeiten und ihre Lebensdauer verlängert wird. Darüber hinaus kann die Schutzschicht den Sensor oder die Sensoren vor elektromagnetischen Wellen schützen, die in der Betriebsumgebung erzeugt werden und möglicherweise die Sensorwerte beeinflussen. Die Schutzschicht trägt somit zur Gesamtfunktionalität und Zuverlässigkeit der Sonde bei, indem sie die Stabilität der Signalverarbeitung und -weiterleitung gewährleistet.

Die Leiterplatte kann auf beiden Seiten ein oder mehrere Sensorelement(e) umfassen. Das bedeutet, dass sowohl die Ober- als auch die Unterseite der Leiterplatte genutzt werden. Beispielsweise können durch Ätzverfahren spezifische Strukturen erzeugt werden, die als Sensorelemente fungieren. Diese geätzten Schichten ermöglichen die Integration von Sensorelementen direkt in die Leiterplatte, wodurch Platz gespart und die Komplexität des Designs reduziert wird. Ferner können andere Synthesemethoden für die Herstellung des Sensorelements bzw. der Sensorelemente verwendet werden. Die Verwendung beider Seiten erhöht die Fläche für die Platzierung von Sensorelementen, was die Sensordichte erhöht und die Leistungsfähigkeit des Systems verbessert. Durch die doppelseitige Ätzung können komplexe Sensoranordnungen realisiert werden, die eine höhere Empfindlichkeit und Genauigkeit bei der Messung verschiedener Parameter ermöglichen. Diese Technik ist vorteilhaft in Anwendungen, bei denen der Platz begrenzt ist oder eine hohe Integration von Funktionen erforderlich ist, wie beispielsweise bei der Ölmessung in einem Ölausdehnungsgefäß eines Öltransformators.

Die Sonde kann des Weiteren Folgendes umfassen: ein mechanisches Gewinde, das an der Leiterplatte angebracht ist, und eine SubMiniature Version A, SMA, / Hochfrequenz, RF, - Schnittstelle, die mit der Leiterplatte elektrisch verbunden und für einen elektrischen Anschluss der Sonde eingerichtet ist. Die Sonde kann darüber hinaus ein mechanisches Gewinde umfassen, das an der Leiterplatte befestigt ist, sowie eine SubMiniature Version A (SMA) oder Hochfrequenz (RF)-Schnittstelle, die elektrisch mit der Leiterplatte verbunden ist und für den elektrischen Anschluss der Sonde ausgelegt ist. Das mechanische Gewinde ermöglicht eine stabile und sichere Befestigung der Sonde an einem Gehäuse oder einer Montagevorrichtung, was insbesondere in industriellen Anwendungen wichtig ist, um Vibrationen oder mechanische Belastungen zu minimieren und die Position der Sonde präzise zu halten. Das mechanisches Gewinde ermöglicht eine öldichte Verbindung und kann eine Gummidichtung umfassen. Die SMA- oder RF-Schnittstelle dient als standardisierte Verbindung für Hochfrequenzsignale und gewährleistet eine effiziente und zuverlässige Übertragung elektrischer Signale zwischen der Sonde und externen Geräten oder Systemen. Durch die elektrische Verbindung mit der Leiterplatte können die von den Sensorelementen erfassten Daten direkt über die SMA/RF-Schnittstelle übertragen werden. Die Kombination aus mechanischem Gewinde und SMA/RF-Schnittstelle erhöht die Funktionalität der Sonde, indem sie sowohl eine robuste mechanische Befestigung als auch eine hochwertige elektrische Verbindung bietet. Dies trägt dazu bei, die Leistungsfähigkeit, Zuverlässigkeit und Langlebigkeit der Sonde zu steigern, indem es eine sichere Montage und eine effiziente Datenkommunikation ermöglicht.

Die Sonde kann ferner Folgendes umfassen: eine Vielzahl von Leiterplatten mit jeweils einem oder mehreren Sensorelement(en), und einen Multiplexer und/oder Demultiplexer, der zur Steuerung eines Signalflusses von und zu den Sensorelementen der Vielzahl von Leiterplatten konfiguriert ist. Beispielsweise können durch Ätzverfahren spezifische Strukturen erzeugt werden, die als Sensorelemente fungieren. Ferner kann auf jeder Leiterplatte ein Sensor vorgesehen sein. Diese Vielzahl von Leiterplatten ermöglicht es, die Anzahl der Sensorelemente zu erhöhen, was die Empfindlichkeit und Genauigkeit der Messungen verbessert. Der in die Sonde integrierte Multiplexer und/oder Demultiplexer ist so konfiguriert, dass er den Signalfluss von und zu den Sensorelementen dieser verschiedenen Leiterplatten steuert. Dies bedeutet, dass er die Signale von mehreren Sensorelementen sammeln und an eine Verarbeitungseinheit weiterleiten kann oder Steuersignale an spezifische Sensorelemente sendet. Durch die koordinierte Steuerung des Signalflusses zwischen den zahlreichen Sensorelementen und der zentralen Verarbeitungseinheit wird eine effiziente Datenverwaltung ermöglicht. Diese Architektur erlaubt es, komplexe Messaufgaben zu bewältigen und die Leistungsfähigkeit der Sonde zu steigern, indem sie eine höhere Datenrate und verbesserte Signalqualität gewährleistet.

Die eingangs gestellte Aufgabe wird auch durch ein Messsystem gelöst, welches eine vorstehend beschriebene Sonde und eine Signaleinheit umfasst. Die Signaleinheit ist dazu eingerichtet, ein elektrisches Signal an den Multiplexer und/oder Demultiplexer zu senden und/oder ein elektrisches Signal von dem Multiplexer und/oder Demultiplexer zu empfangen. Das Messsystem besteht somit aus einer der zuvor beschriebenen Sonden und einer Signaleinheit. Die Signaleinheit ist dafür ausgelegt, elektrische Signale an den auf der Leiterplatte der Sonde integrierten Multiplexer und/oder Demultiplexer zu senden und von diesem zu empfangen. Der Multiplexer/Demultiplexer steuert den Signalfluss zwischen den Sensorelementen der Sonde und der Signaleinheit. Durch das Senden von Signalen kann die Signaleinheit bestimmte Sensorelemente aktivieren, steuern oder deren Daten abrufen. Die empfangenen Signale von den Sensorelementen werden über den Multiplexer gebündelt und an die Signaleinheit übertragen, wo sie verarbeitet, analysiert oder gespeichert werden können. Dieses Zusammenspiel ermöglicht eine effiziente Kommunikation und Steuerung innerhalb des Messsystems, wodurch präzise Messungen und Echtzeitüberwachung der relevanten Parameter gewährleistet werden. Die Signaleinheit fungiert dabei als zentrales Kontroll- und Verarbeitungselement, das die Daten der Sonde nutzt, um den Zustand des überwachten Betriebsmittels zu bewerten und gegebenenfalls entsprechende Maßnahmen einzuleiten. Bei der Signaleinheit kann es sich insbesondere um eine Signalerzeugungseinheit handeln.

Die oben beschriebenen Aspekte und Varianten können kombiniert werden, ohne dass dies explizit beschrieben ist. Jede der beschriebenen Ausgestaltungsvarianten ist somit optional zu jeder Ausgestaltungsvariante oder Kombinationen davon zu sehen. Die vorliegende Offenbarung ist somit nicht auf die einzelnen Ausgestaltungen und Varianten in der beschriebenen Reihenfolge oder einer bestimmten Kombination der Aspekte und Ausgestaltungsvarianten beschränkt.

### KURZE BESCHREIBUNG DER ZEICHNUNGEN

Weitere Vorteile, Einzelheiten und Merkmale der hier beschriebenen Vorrichtungen und Verfahren ergeben sich aus der nachfolgenden Beschreibung von Ausführungsbeispielen und den Figuren.
- Fig. 1: zeigt eine schematische Darstellung eines ersten Ausführungsbeispiels einer Sonde für ein zumindest teilweise in Öl befindliches elektrisches Betriebsmittel;
- Fig. 2: zeigt eine schematische Darstellung eines zweiten Ausführungsbeispiels einer Sonde für ein zumindest teilweise in Öl befindliches elektrisches Betriebsmittel; und
- Fig. 3: zeigt eine schematische Darstellung eines Ausführungsbeispiels eines Öltransformators mit einer Vielzahl von Sonden.

### DETAILLIERTE BESCHREIBUNG

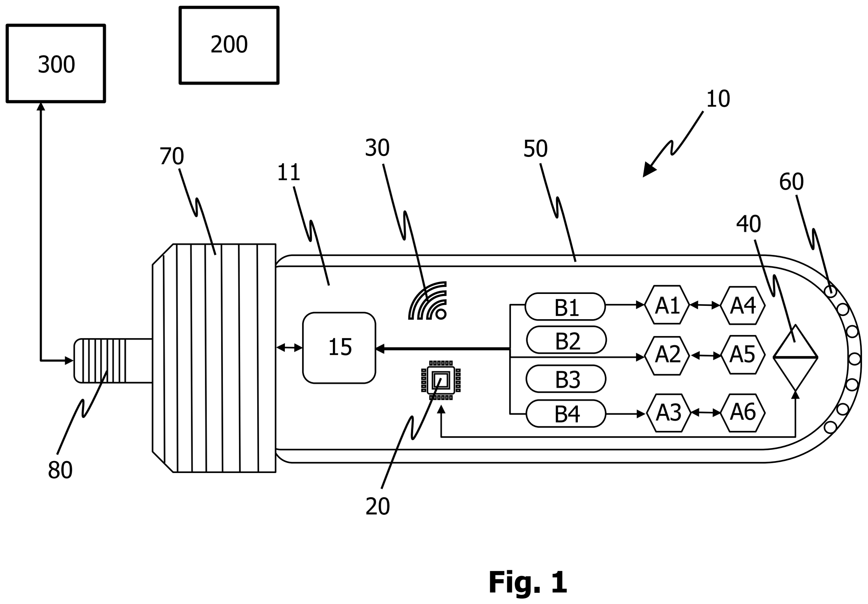

Die Fig. 1 zeigt eine schematische Darstellung eines ersten Ausführungsbeispiels einer Sonde 10 für ein zumindest teilweise in Öl befindliches elektrisches Betriebsmittel.

Die Sonde 10 umfasst eine Leiterplatte 11, an der ein mechanisches Gewinde 70 angebracht ist. Eine SMA/RF-Schnittstelle 80 ist mit der Leiterplatte 11 elektrisch verbunden und ermöglicht einen elektrischen Anschluss der Sonde 10 an eine Signaleinheit 300, beispielsweise einen Signalgenerator.

Auf der Leiterplatte 11 befinden sich mehrere Sensorelemente A1 bis A6. Zudem ist auf der Leiterplatte 11 ein Multiplexer/Demultiplexer 15 integriert, der zur Steuerung des Signalflusses von und zu den Sensorelementen A1 bis A6 konfiguriert ist. Die Sensorelemente A1 bis A6 weisen verschiedene geometrische Formen auf und die Anzahl der Sensorelemente ist nicht auf sechs beschränkt. Die Sensorelemente A1 bis A6 sind miteinander sowie mit dem Multiplexer/Demultiplexer 15 elektrisch verbunden und können über ein Telekommunikationsprotokoll miteinander kommunizieren.

Die Sensorelemente A1 bis A6 umfassen chemische Katalysatoren und Mikroreaktoren. Die chemischen Katalysatoren sind so konfiguriert, dass sie mit einem im Öl gelösten Gas reagieren, um ein elektrisches Signal zu erzeugen, das als Indikator für die Anwesenheit und/oder die Konzentration des Gases dient. Die Mikroreaktoren sind so konfiguriert, dass sie mit einem im Öl gelösten Gas interagieren, um ein elektrisches Signal zu erzeugen, das auf die Anwesenheit und/oder die Konzentration des Gases hinweist.

Der Multiplexer/Demultiplexer 15 ist zum sequenziellen oder gleichzeitigen Senden und/oder Empfangen von elektrischen Signalen zu bzw. von den Sensorelementen A1 bis A6 eingerichtet, wodurch Leitungen eingespart werden können.

Die Sonde 10 kann weitere optionale Elemente umfassen, die in der Abbildung 1 mit dem Bezugszeichen 20 dargestellt sind. Dabei kann es sich um eine oder mehrere der folgenden Komponenten handeln: eine eingebettete Einheit, eine anwendungsspezifisch integrierte Schaltung (ASIC), eine System-on-a-Chip (SoC) elektronische Einheit, einen Mikrocontroller (MCU), einen Digital-Analog-Wandler (DAC) und einen Analog-Digital-Wandler (ADC). Diese Elemente sind auf der Leiterplatte 11 in elektrischer Verbindung mit dem Multiplexer/Demultiplexer 15 und den Sensorelementen A1 bis A6 angeordnet.

Als weiteres optionales Element kann ein Kommunikationsmodul 30 in Kommunikationsverbindung mit einer oder mehreren der eingebetteten Einheit, dem ASIC, der SoC-elektronischen Einheit, dem MCU, dem DAC und dem ADC auf der Leiterplatte 11 vorgesehen sein. Das Kommunikationsmodul 30 ist dazu eingerichtet, Messwerte der Sensorelemente A1 bis A6 an eine externe Empfangseinheit 200 zu senden. In der externen Empfangseinheit 200, beispielsweise einem Cloud-Server, können die empfangenen Messwerte weiterverarbeitet werden.

Ferner kann die Sonde 10 einen oder mehrere Sensoren B1 bis B4 umfassen, die auf der Leiterplatte 11 vorgesehen und mit dem Multiplexer/Demultiplexer 15 elektrisch verbunden sind und dazu eingerichtet sind, eine Temperatur, Viskosität, Vibration und/oder den Durchfluss von Öl zu messen. Die Anzahl der Sensoren B1 bis B4 ist nicht auf vier beschränkt.

Des Weiteren kann die Sonde 10 auf der Leiterplatte 11 eine Temperatursteuerungseinheit 40 umfassen, die dazu eingerichtet ist, die Temperatur der Sensorelemente A1 bis A6 zu steuern. Dazu kann die Temperatursteuerungseinheit 40 mit dem MCU 20 verbunden sein und von diesem Steuerungssignale empfangen.

Ein Gehäuse 50 umgibt die Leiterplatte 11. In dem abgedichteten Gehäuse 50 ist ein Filterabschnitt 60 integriert. Der Filterabschnitt 60 besteht aus einer Membran, die dazu eingerichtet ist, Partikel aus dem Öl zu filtern und/oder Gase aus dem Öl zu extrahieren.

Die Leiterplatte 11 kann auf beiden Seiten Sensorelemente A1 bis A6 umfassen.

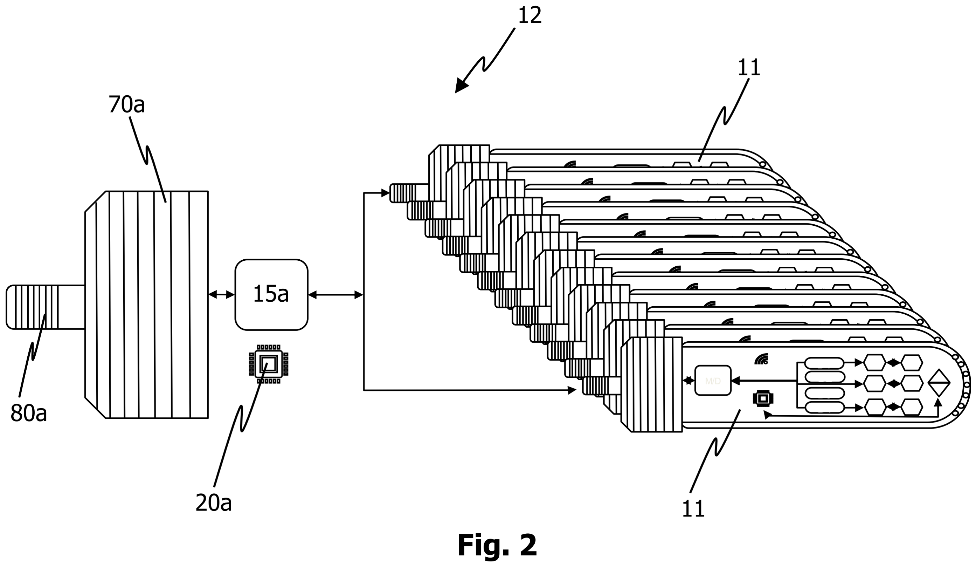

Die Fig. 2 zeigt eine schematische Darstellung eines zweiten Ausführungsbeispiels einer Sonde 12 für ein zumindest teilweise in Öl befindliches elektrisches Betriebsmittel.

Die Sonde 12 umfasst eine Vielzahl von Leiterplatten 11, die jeweils Sensorelemente aufweisen. Zudem ist ein Multiplexer/Demultiplexer 15a integriert, der zur Steuerung des Signalflusses von und zu den Sensorelementen der verschiedenen Leiterplatten 11 konfiguriert ist. Dabei handelt es sich bei den Leiterplatten 11 um gestapelte Leiterplatten, die den in Fig. 1 gezeigten Leiterplatten 11 entsprechen können.

Ferner umfasst die Sonde 12 eine Einheit 20a, die aus einem oder mehreren der folgenden Elemente bestehen kann: einer eingebetteten Einheit, einem ASIC, einer System-on-a-Chip (SoC) elektronischen Einheit, einem Mikrocontroller (MCU), einem Digital-Analog-Wandler (DAC) und/oder einem Analog-Digital-Wandler (ADC). Diese Elemente stehen in Kommunikationsverbindung mit dem Multiplexer/Demultiplexer 15a.

Des Weiteren verfügt die Sonde 12 über ein mechanisches Gewinde 70a sowie eine SMA/RF-Schnittstelle 80a, die einen elektrischen Anschluss der Sonde 12 an eine Signaleinheit 300, beispielsweise einen Signalgenerator, ermöglichen.

Dieses zweite Ausführungsbeispiel der Sonde 12 erweitert die Funktionalität durch die Nutzung mehrerer gestapelter Leiterplatten, wodurch die Anzahl der integrierten Sensorelemente erhöht wird. Der Multiplexer/Demultiplexer 15a ermöglicht eine effiziente Steuerung des Signalflusses zwischen den Sensorelementen und den elektronischen Einheiten, was die Gesamtleistung und Genauigkeit der Messungen verbessert. Die Kommunikationsverbindung zwischen den elektronischen Einheiten und dem Multiplexer/Demultiplexer stellt sicher, dass die erfassten Daten präzise verarbeitet und an die Signaleinheit 300 weitergeleitet werden können. Durch die Integration des mechanischen Gewindes 70a und der SMA/RF-Schnittstelle 80a wird eine robuste und zuverlässige Verbindung zur externen Signaleinheit gewährleistet, was die Flexibilität und Einsatzmöglichkeiten der Sonde 12 in verschiedenen Anwendungen erhöht.

Insgesamt bietet dieses zweite Ausführungsbeispiel eine verbesserte Skalierbarkeit und Funktionalität, indem es die Anzahl der Sensorelemente erweitert und eine effiziente Signalverarbeitung sowie zuverlässige Kommunikationswege integriert. Dies führt zu einer umfassenderen Überwachung und präziseren Messungen des Zustands des ölgetauchten elektrischen Betriebsmittels.

Die in der Fig. 2 gezeigten mechanischen Gewinde und SMA/RF-Schnittstellen der Vielzahl von Leiterplatten 11 können durch andere elektrische Anschlüsse zur Verbindung mit dem Multiplexer/Demultiplexer 15a ersetzt werden.

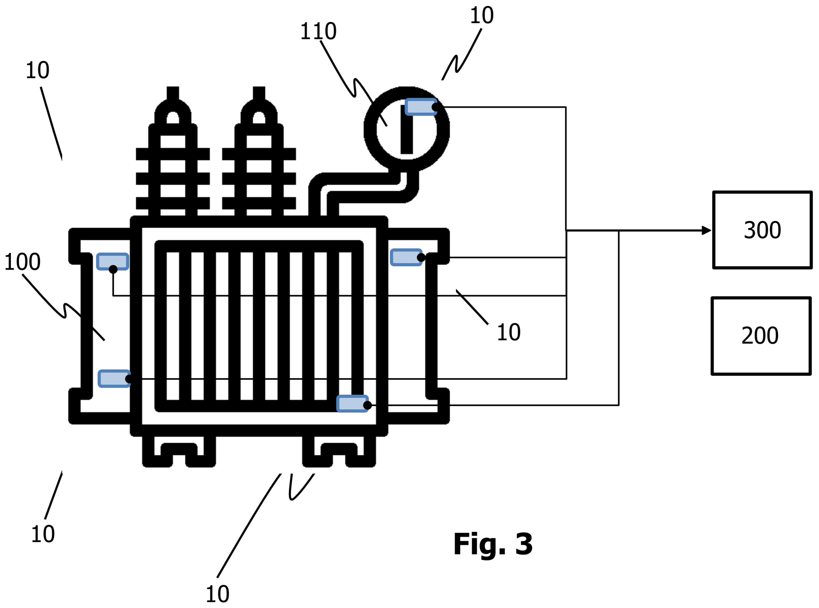

Die Fig. 3 zeigt eine schematische Darstellung eines Ausführungsbeispiels eines Öltransformators mit einer Vielzahl von Sonden.

Bei den Sonden kann es sich sowohl um die in Fig. 1 dargestellte Sonde 10 als auch um die in Fig. 2 gezeigte Sonde 12 handeln. Eine Sonde 10 ist in einem Ölausdehnungsgefäß 110 des Öltransformators angeordnet. Vier weitere Sonden 19 befinden sich in einer Ölwanne 100 des Öltransformators, in der sich der Transformatorkern befindet. Die Sonden 10 sind mit einer Signaleinheit 300 verbunden, die dazu eingerichtet ist, elektrische Signale an die Multiplexer/Demultiplexer in den jeweiligen Sonden 10 zu senden bzw. von diesen zu empfangen.

Darüber hinaus können Kommunikationsmodule in den Sonden 10 die Messwerte drahtlos an eine externe Empfangseinheit 200 senden, wo sie weiterverarbeitet werden.

Gemäß einer Weiterentwicklung des Ausführungsbeispiels in Fig. 3 können mehrere Öltransformatoren mit installierten Sonden 10 miteinander vernetzt sein und/oder ihre Messwerte an eine zentrale Verarbeitungseinheit senden.

Diese Vernetzung ermöglicht eine umfassende Überwachung und Analyse der Zustände mehrerer Transformatoren gleichzeitig. Durch die zentrale Verarbeitungseinheit können die gesammelten Daten effizient ausgewertet und analysiert werden, was eine frühzeitige Erkennung von Anomalien und eine proaktive Wartung der Transformatoren erleichtert. Die Integration verschiedener Sonden in einem vernetzten System erhöht die Genauigkeit und Zuverlässigkeit der Messungen, da unterschiedliche Parameter gleichzeitig erfasst und korreliert werden können. Somit trägt dieses System zur Optimierung der Betriebsbedingungen und zur Verlängerung der Lebensdauer der Öltransformatoren bei.

Zusammenfassend bietet das beschriebene Ausführungsbeispiel der Fig. 3 eine effektive Lösung zur kontinuierlichen Überwachung des Zustands von ölgetauchten elektrischen Betriebsmitteln. Die Kombination aus mehreren Sonden, Kommunikationsmodulen und einer zentralen Verarbeitungseinheit ermöglicht eine detaillierte und zuverlässige Analyse der Ölqualität und der betrieblichen Gesundheit der Transformatoren.

Das Ausführungsbeispiel der Fig. 3 ist nicht auf Öltransformatoren beschränkt und der/die Öltransformator(en) können durch folgende elektrische Betriebsmittel ersetzt werden: einen Ölschalter, eine ölgefüllte Schaltanlage, einen ölgefüllten Strom- und Spannungswandler, einen ölgefüllten Kondensator, eine ölgefüllte Drosselspule, einen ölgefüllten Reaktor, eine ölgefüllte Durchführung, ein ölisoliertes Kabel, einen ölgefüllten Laststufenschalter, einen ölgefüllten Motor, einen ölgefüllten Generator, einen ölgefüllten Leistungssteller, einen ölgefüllten Thyristor oder einen ölgefüllten Lichtbogenofen handeln.

In den vorgestellten Beispielen sind unterschiedliche Merkmale und Funktionen der vorliegenden Offenbarung getrennt voneinander sowie in bestimmten Kombinationen beschrieben worden. Es versteht sich jedoch, dass viele dieser Merkmale und Funktionen, wo dies nicht explizit ausgeschlossen ist, miteinander frei kombinierbar sind.

## Patentansprüche

1. Sonde (10, 12) für ein zumindest teilweise in Öl befindliches elektrisches Betriebsmittel, umfassend:
eine Leiterplatte (11) mit einem oder mehreren Sensorelement(en) (A1 - A6), und
einen auf der Leiterplatte integrierten Multiplexer und/oder Demultiplexer (15), der zur Steuerung eines Signalflusses von und zu den Sensorelementen (A1 - A6) konfiguriert ist.

2. Sonde (10, 12) nach Anspruch 1, wobei
die Sensorelemente (A1 - A6) durch geätzte Schichten gebildet werden, und/oder chemische Katalysatoren und/oder Mikroreaktoren und/oder chemische Zusammensetzungen umfassen, wobei
die chemischen Katalysatoren so konfiguriert sind, dass sie mit einem im Öl gelösten Gas reagieren, um ein elektrisches Signal zu erzeugen, das ein Indikator für eine Anwesenheit und/oder eine Konzentration des Gases ist, und
die Mikroreaktoren so konfiguriert sind, dass sie mit einem im Öl gelösten Gas interagieren, um ein elektrisches Signal zu erzeugen, das auf eine Anwesenheit und/oder eine Konzentration des Gases hinweist.

3. Sonde (10, 12) nach einem der vorhergehenden Ansprüche, wobei
die Sensorelemente (A1 - A6) verschiedene geometrische Formen aufweisen; und/oder
die Sensorelemente (A1 - A6) dazu eingerichtet sind, miteinander über ein Telekommunikationsprotokoll zu kommunizieren.

4. Sonde (10, 12) nach einem der vorhergehenden Ansprüche, wobei
der Multiplexer und/oder Demultiplexer (15) zum sequenziellen oder gleichzeitigen Senden und/oder Empfangen von elektrischen Signalen zu bzw. von den Sensorelementen (A1 - A6) eingerichtet ist.

5. Sonde (10, 12) nach einem der vorhergehenden Ansprüche, ferner umfassend eine eingebettete Einheit (20), eine anwendungsspezifisch integrierte Schaltung, ASIC, eine System-on-a-Chip, SoC, elektronische Einheit, einen Mikrocontroller, MCU, einen Digital-Analog-Wandler, DAC, und/oder einen Analog-Digital-Wandler, ADC, der/die auf der Leiterplatte (11) in elektrischer Verbindung mit dem Multiplexer und/oder Demultiplexer (15) und den Sensorelementen (A1 - A6) angeordnet ist/sind.

6. Sonde (10, 12) nach Anspruch 5, ferner umfassend
ein Kommunikationsmodul (30) in Kommunikationsverbindung mit einem der eingebetteten Einheit (20), der anwendungsspezifisch integrierten Schaltung, ASIC, der System-on-a-Chip, SoC, elektronischen Einheit, dem Mikrocontroller, MCU, dem Digital-Analog-Wandler, DAC, und/oder dem Analog-Digital-Wandler, ADC, und das dazu eingerichtet, Messwerte der Sensorelemente (A1 - A6) drahtlos an eine externe Empfangseinheit (200) zu senden.

7. Sonde (10, 12) nach einem der vorhergehenden Ansprüche, ferner umfassend
einen Sensor (B1 - B4), der mit dem Multiplexer und/oder Demultiplexer (15) elektrisch verbunden und dazu eingerichtet ist, eine Temperatur, eine Viskosität, eine Vibration und/oder einen Durchfluss des Öls zu messen.

8. Sonde (10, 12) nach einem der vorhergehenden Ansprüche, ferner umfassend
eine Temperatursteuerungseinheit (40), die dazu eingerichtet ist, eine Temperatur der Sensorelemente (A1 - A6) zu steuern.

9. Sonde (10, 12) nach einem der vorhergehenden Ansprüche, ferner umfassend
einen Filterabschnitt (60), insbesondere eine Membran, der dazu eingerichtet ist, Partikel aus dem Öl zu filtern und/oder Gas aus dem Öl zu extrahieren.

10. Sonde (10, 12) nach Anspruch 9, ferner umfassend
ein die Leiterplatte (11) umgebendes Gehäuse (50), wobei der Filterabschnitt (60) in dem Gehäuse (50) integriert ist.

11. Sonde (10, 12) nach einem der vorhergehenden Ansprüche, ferner umfassend
eine Schutzschicht, die zumindest den Multiplexer und/oder Demultiplexer (15) umgibt.

12. Sonde (10, 12) nach einem der vorhergehenden Ansprüche, wobei
die Leiterplatte (11) auf beiden Seiten ein oder mehrere Sensorelement(e) (A1 - A6) umfasst.

13. Sonde (10, 12) nach einem der vorhergehenden Ansprüche, ferner umfassend
ein mechanisches Gewinde (70), das an der Leiterplatte (11) angebracht ist, und
eine SubMiniature Version A, SMA, / Hochfrequenz, RF, -Schnittstelle (80), die mit der Leiterplatte (11) elektrisch verbunden und für einen elektrischen Anschluss der Sonde (10, 12) eingerichtet ist.

14. Sonde (12) nach einem der vorhergehenden Ansprüche, ferner umfassend
eine Vielzahl von Leiterplatten (11) jeweils mit einem oder mehreren Sensorelement(en) (A1 - A6), und
einen Multiplexer und/oder Demultiplexer (15a), der zur Steuerung eines Signalflusses von und zu den Sensorelementen (A1 - A6) der Vielzahl von Leiterplatten (11) konfiguriert ist.

15. Messsystem umfassend
eine Sonde (10, 12) nach einem der vorhergehenden Ansprüche und
eine Signaleinheit (300), die dazu eingerichtet ist, ein elektrisches Signal an den Multiplexer und/oder Demultiplexer (15; 15a) zu senden und/oder ein elektrisches Signal von dem Multiplexer und/oder Demultiplexer (15; 15a) zu empfangen.
